# EUROPÄISCHE PATENTANMELDUNG

(11) **EP 2 505 180 A1**
(43) Veröffentlichungstag der Anmeldung: **03.10.2012**
(21) Anmeldenummer: 11160802.2
(22) Anmeldetag: 01.04.2011
(51) Int. Cl.: A61K 8/02, A61K 8/06, A61K 8/33, A61K 8/34, A61K 8/37, A61K 8/44, A61K 8/46, A61K 8/49, A61K 8/60, A61Q 1/14, A61Q 5/02, A61Q 19/10

(54) **Feinteilige Emulsionen enthaltend Mikroemulsionen**

(71) Anmelder: Cognis IP Management GmbH, 40589 Düsseldorf (DE)
(72) Erfinder: Hloucha, Matthias, 50677 Köln (DE); Duerrschmidt Olga, Georgievna, 40724 Hilden (DE); Kawa, Rolf, 40789 Monheim (DE); Strauss, Gabriele, 40589 Düsseldorf (DE); Schulte, Petra, 50733 Köln (DE); Menzer, Jasmin, 40789 Monheim (DE); Stork, Anja, 50733 Köln (DE)

(57) **Zusammenfassung**

Vorgeschlagen werden Mikroemulsionen enthaltend:
a1) eine Mischung aus Alkyl(oligo)glycosiden,
a2) mindestens ein Cotensid,
a3) mindestens ein anionisches Tensid, das sich auszeichnet durch Säurefunktionen, die ganz oder teilweise neutralisiert vorliegen können,
a4) eine organische Ölphase ausgenommen alkoxylierte Verbindungen,
a5) gegebenenfalls Fettalkohole ausgenommen alkoxylierte Fettalkohole,
a6) gegebenenfalls kosmetische Zusatzstoffe,
a7) Wasser add 100 Gew.-%.

Sowie feinteilige Emulsionen die neben einer weiteren Ölphase und Konservierungsmittel diese Mikroemulsionen enthalten. Die feinteilige Emulsion wird bevorzugt durch Salz stabilisiert.
Eingesetzt werden die Mikroemulsion und die feinteilige Emulsion als kosmetisches Reinigungsmittel in Tüchern, Duschgelen, Handwaschmitteln, Gesichtsreinigern, make-up-Entfernern, Badezubereitungen, Babypflegeprodukten, Shampoo oder als Tränkungsmedium für Tücher.

## Beschreibung

Die Erfindung befindet sich auf dem Gebiet der kosmetischen Mittel, die in Form feinteiliger Emulsionen auf Basis ölhaltiger Mikroemulsionen vorliegen und betrifft weiterhin ein Verfahren zur Herstellung solcher Emulsionen sowie deren Verwendung u.a. auf Flächengebilden.

Es ist bekannt, dass Öl-in-Wasser-Emulsionen, die mit nichtionischen Emulgatoren hergestellt sind, beim Erwärmen häufig eine Phaseninversion erleiden, d. h., dass bei höheren Temperaturen die äußere, wässerige Phase zur inneren Phase werden kann. Dieser Vorgang ist in der Regel reversibel, so dass sich beim Abkühlen wieder der ursprüngliche Emulsionstyp zurückbildet. Emulsionen, die oberhalb der Phaseninversionstemperatur hergestellt wurden, weisen im Allgemeinen eine niedrige Viskosität und hohe Lagerstabilität auf.

An kosmetische Reinigungszubereitungen werden vom Verbraucher immer höhere Ansprüche gestellt. So sollen derartige Zubereitungen nicht nur über hervorragendes Schaumverhalten und gute Reinigungsleistung verfügen, sondern auch Haut und Haar pflegen und konditionieren. Bei den pflegenden Wirkstoffen oder Hautkonditioniermitteln handelt es sich meistens um ölige Substanzen. Ölhaltige wässrige Tensidformulierungen zeigen üblicherweise ein schlechtes Schaumverhalten. Hierunter ist sowohl ein schlechtes Anschäumen als auch eine niedrige maximale Schaummenge zu verstehen. Daher werden sehr häufig ethoxylierte Verbindungen, wie z.B. Natriumlaurethsulfat, als Tensidkomponente verwendet. Da diese Verbindungen jedoch immer mehr in Verruf geraten, besteht eine große Nachfrage nach Formulierungen, die frei von alkoxylierten und speziell ethoxylierten Verbindungen sind. Bei den alkoxylierten Verbindungen handelt es sich um Substanzen, die aus der Petrochemie stammen und mit klassischen chemischen Verfahren hergestellt werden. Gerade in der Kosmetik ist die Nachfrage nach Substanzen, die aus nachwachsenden Rohstoffen gewonnen werden können besonders groß. Durch den Einsatz von alkoxylierten Tensiden, meist Alkylethersulfaten, können bei der Anwendung der kosmetischen Mittel Hautirritationen auftreten und außerdem häufen sich die Rufe nach "grünen Kosmetika", die frei von alkoxylierten Verbindungen sind. Für diese Einschränkungen bezüglich der Tenside gibt es bisher keine befriedigende Lösung, um Reinigungsmittel mit guten Schaumeigenschaften bereitzustellen. In der WO 20081155075 werden kosmetische Zubereitungen beschrieben, die neben nicht-alkoxylierten Tensiden eine Mikroemulsion und mindestens ein kationische Polymer enthalten. Diese Zubereitungen werden eingesetzt als Konditionierungsmittel in Shampoo und Haarbehandlungsmitteln. Zur besseren Konditionierwirkung muss zwingend ein kationisches Polymer eingesetzt werden.
Weiterhin besteht der Wunsch beim Verbraucher bzw. beim Endprodukthersteller feinteilige Emulsionen zu erhalten, die auf Zusatzstoffe zur Erhöhung der Lagerstabilität verzichten, welche potentiell zu Hautirritationen oder Allergien führen können.

Außerdem erfreuen sich transparente tensidische Zubereitungen größter Beliebtheit, wobei es sich als schwierig erwiesen hat, diese Transparenz auch bei Zusatz von Ölkörpern aufrecht zu erhalten.
So beschreibt die WO 98140044 wässrige Zubereitungen wasserlöslicher Tenside, die Lipid-Tensid-Mischmizellen mit einer mittleren Teilchengröße von unter 500 nm aufweisen und dabei weiß-bläulich erscheinen. Gegenstand der WO 98115255 sind Mikroemulsionsgele vom Typ Öl-in-Wasser, bei denen die Öltröpfchen in der Wasserphase durch assoziative Verdicker stabilisiert werden.

Die Verwendung von Reinigungs- und Hautpflegemitteln direkt auf Flächengebilden wie Feuchttüchern, Wipes, Kosmetiktücher oder ähnlichem haben eine breite Anwendung speziell in der Pflege von Babys aber auch in der Körperpflege Erwachsener gefunden.
Es besteht jedoch weiterhin ein hoher Bedarf an Pflegeprodukten, die auf Flächengebilden ein Gefühl der Weichheit ergeben, hohe Reinigungswirkung zeigen und ein gutes Hautgefühl hinterlassen.

Die WO 021072052 (EP1372599) beschreibt Mikroemulsionen zum Einsatz auf Wipes, die jedoch zur besseren Schaumeigenschaft ethoxylierte Verbindungen enthalten. Desweiteren enthalten diese Emulsionen Monoester von Glycerin, die ebenfalls Nachteile hinsichtlich der Stabilität bringen bei der Herstellung feinteiliger transparenter Emulsionen und bei der Zugabe von Zusatzstoffen wie Parfum.

In EP 1813251 werden kaltherstellbare mikroemulsionsartige Konzentrate beschrieben, die PEG-freie Emulgatormischungen aus nichtionischen Emulgator und einem mindestens eine Säurefunktion enthaltenden Emulgator enthalten, sowie ein Cotensid und ein oder mehrere Öle. Der Gesamtwasserphasengehalt ist größer oder gleich 10 bis 70 Gew. %. Nachteilig an diesem umfangreich beschriebenen Konzentrat ist, dass die Ölphase sowohl alkoxylierte Alkohole oder alkoxylierte organische Säuren oder Mischungen daraus enthalten als auch Silikonöle.

Die Aufgabe der vorliegenden Erfindung hat somit darin bestanden, kosmetische Mittel bereit zu stellen, die bei Vermeidung von alkoxylierten Inhaltsstoffen mindestens gleich gute Solubilisierungs- und Reinigungseigenschaften aufweisen wie Zusammensetzungen auf Basis von alkoxylierten Inhaltsstoffen und deren Schaumverhalten dem von alkoxylierten Inhaltstoffen enthaltenden Zubereitungen entspricht oder bestenfalls diese sogar noch übertrifft. Die kosmetischen Mittel sollten eine schnelle Benetzung von Flächengebilden möglich machen also möglichst dünnflüssig sein und gleichzeitig eine geringe Klebrigkeit der behandelten Flächengebilde ergeben ohne silikonhaltige Verbindungen zu enthalten. Die feinteilige Emulsion sollte frei von alkoxylierten Verbindungen, frei von silikonhaltigen Verbindungen und frei von Monoestern des Glycerins sein und dennoch den hohen Anforderungen an kosmetischen Reinigungsmitteln entsprechen die als Tränklösung oder als sprühbare Emulsion verwendbar sind.
Die verdünnte feinteilige Emulsion soll eine hohe Stabilität zeigen.

Es war für den Fachmann nicht vorauszusehen, dass die Aufgabe gelöst werden konnte durch eine neuartige Mikroemulsion bzw. eine feinteilige Emulsion, die diese Mikroemulsion enthält, welche sich durch die Mischung von Tensid, Cotensid, Ölphase und gegebenenfalls Fettalkohol auszeichnet.

Durch die überraschend einfache Kombination von den erfindungsgemäßen Tensiden, Cotensiden, Ölphasen und gegebenenfalls weiteren Zusatzstoffen wie Fettalkoholen lassen sich transparente, dünnflüssige Mikroemulsionen und feinteilige Emulsionen herstellen, die eine verminderte bzw. keinerlei objektive als auch subjektive Klebrigkeit aufweisen.

Gegenstand der Erfindung betrifft eine Mikroemulsion enthaltend:
a1) eine Mischung aus Alkyl(oligo)glycosiden,
a2) mindestens ein Cotensid,
a3) mindestens ein anionisches Tensid, das sich auszeichnet durch Säurefunktionen, die ganz oder teilweise neutralisiert vorliegen können,
a4) eine organische Ölphase, ausgenommen alkoxylierte Verbindungen,
a5) gegebenenfalls Fettalkohole, ausgenommen alkoxylierte Fettalkohole,
a6) gegebenenfalls kosmetische Zusatzstoffe,
a7) Wasser add 100 Gew.%.

Die erfindungsgemäße Mikroemulsion kann in der Form oder als verdünnte Lösung zum Einsatz kommen. Für die Verwendung in Reinigungsmitteln wie Shampoo, Babypflegeprodukten, Hautreinigern kann die Mikroemulsion direkt eingesetzt werden. Für den Einsatz als Tränkungsmittel für Flächengebilde ist eine Verdünnung mit Wasser bevorzugt.

In einer bevorzugten Ausführungsform enthält die Mikroemulsion
a1) 10 - 30 Gew.-% einer Mischung aus Alkyl(oligo)glycosiden,
a2) 2 -15 Gew.-% Cotenside,
a3) 0,2 - 4 Gew.-% mindestens eines anionischen Tensides,
a4) 10 - 30 Gew.% organische Ölphase ausgenommen alkoxylierte Verbindungen,
a5) 0 - 4 Gew.-% Fettalkohole ausgenommen alkoxylierte Fettalkohole,
a6) 0 - 20 Gew.-% kosmetische Zusatzstoffe,
a7) Wasser add 100 Gew.-%.

In einer weiteren bevorzugten Ausführungsform der Erfindung besteht die Mikroemulsion aus:
a1) 10 - 30 Gew.-% einer Mischung aus Alkyl(oligo)glycosiden,
a2) 2 - 15 Gew.-% Cotenside,
a3) 0,2 - 4 Gew.-% mindestens eines anionischen Tensides,
a4) 10 - 30 Gew.% organische Ölphase ausgenommen alkoxylierte Verbindungen,
a5) 0 - 4 Gew.-% Fettalkohole ausgenommen alkoxylierte Fettalkohole,
a6) 0 - 20 Gew.-% kosmetische Zusatzstoffe,
a7) Wasser add 100 Gew.-%.

In einer besonders bevorzugten Ausführungsform enthält die Mikroemulsion
a1) 15 - 25 Gew.% einer Mischung aus Alkyl(oligo)glycosiden,
a2) 6 - 12 Gew.-% Cotenside,
a3) 0,5 - 3 Gew.-% mindestens eines anionischen Tensides,
a4) 15 - 25 Gew.% organische Ölphase ausgenommen alkoxylierte Verbindungen,
a5) 0 - 4 Gew.-% Fettalkohole ausgenommen alkoxylierte Fettalkohole,
a6) 0 - 15 Gew.-% kosmetische Zusatzstoffe,
a7) Wasser add 100 Gew.-%.

Durch Einarbeitung der erfindungsgemäßen Mikroemulsion als Komponente (A) der erfindungsgemäßen feinteiligen Emulsion gelingt die transparente und stabile Einarbeitung größerer Mengen von Ölkörpern, der weiteren Ölphase (B) und gegebenenfalls den weiteren Zusatzstoffen (C). Dementsprechend ist ein weiterer bevorzugter Gegenstand der Erfindung eine feinteilige Emulsion, die die erfindungsgemäße Mikroemulsion (A) enthält, sowie gegebenenfalls eine weitere Ölphase (B), ein Konservierungsmittel (C), gegebenenfalls ein Salz als Stabilisator (D) und Wasser (E), bevorzugt demineralisiertes Wasser mit dem auf 100 Gew.-% aufgefüllt wird. Der Anteil an Wasser in der feinteiligen Emulsion liegt bevorzugt bei größer 80%, bezogen auf die gesamte feinteilige Emulsion.

Bei den **Konservierungsmitteln** handelt es sich im Sinne der Erfindung bevorzugt um Verbindungen, die ausgewählt sind aus der Gruppe, die gebildet wird von Benzoesäure und deren Salze, Zitronensäure und deren Salze, Phenoxyethanol, Benzylalkohol, Alkylparabenen, bevorzugt Ethyl-, Methyl- und Propylparaben. Als Konservierungsmittel eignen sich weiterhin beispielsweise Formaldehydlösung, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

Die feinteilige Emulsion enthält bevorzugt:
A) 0,5 - 15 Gew.-% einer erfindungsgemäßen Mikroemulsion enthaltend
   a1) einer Mischung aus Alkyl(oligo)glycosiden,
   a2) mindestens einem Cotensid,
   a3) mindestens einem anionischen Tensid, das sich auszeichnet durch Säurefunktionen, die ganz oder teilweise neutralisiert vorliegen können,
   a4) einer organischen Ölphase ausgenommen alkoxylierte Verbindungen,
   a5) gegebenenfalls Fettalkoholen ausgenommen alkoxylierte Fettalkohole,
   a6) gegebenenfalls kosmetischen Zusatzstoffen,
   a7) Wasser add 100 Gew.-% der Mikroemulsion,
B) 0 - 10 Gew.-% einer weiteren Ölphase,
C) 0,1 - 3 Gew.-% Konservierungsmittel,
D) 0 - 5 Gew.-% Salz,
E) größer 80 Gew.-% Wasser

In einer weiteren bevorzugten Ausführungsform besteht die feinteilige Emulsion aus:
A) 0,5 - 15 Gew.-% einer erfindungsgemäßen Mikroemulsion bestehend aus:
   a1) einer Mischung aus Alkyl(oligo)glycosiden,
   a2) mindestens einem Cotensid,
   a3) mindestens einem anionischen Tensid, das sich auszeichnet durch Säurefunktionen, die ganz oder teilweise neutralisiert vorliegen können,
   a4) einer organischen Ölphase ausgenommen alkoxylierte Verbindungen,
   a5) gegebenenfalls Fettalkoholen ausgenommen alkoxylierte Fettalkohole,
   a6) gegebenenfalls kosmetischen Zusatzstoffen,
   a7) Wasser add 100 Gew.% der Mikroemulsion,
B) 0 - 10 Gew.-% einer weiteren Ölphase,
C) 0,1 - 3 Gew.-% Konservierungsmittel,
D) 0 - 5 Gew.-% Salz,
E) größer 80 Gew.-% Wasser.

Die feinteilige Emulsion enthält besonders bevorzugt:
A) 1 - 10 Gew.-% einer erfindungsgemäßen Mikroemulsion enthaltend
   a1) eine Mischung aus Alkyl(oligo)glycosiden,
   a2) mindestens ein Cotensid,
   a3) mindestens ein anionisches Tensid, das sich auszeichnet durch Säurefunktionen, die ganz oder teilweise neutralisiert vorliegen können,
   a4) eine organische Ölphase ausgenommen alkoxylierte Verbindungen,
   a5) gegebenenfalls Fettalkohole ausgenommen alkoxylierte Fettalkohole,
   a6) gegebenenfalls kosmetischen Zusatzstoffen,
   a7) Wasser add 100 Gew.-% der Mikroemulsion,
B) 0 - 5 Gew.-% einer weiteren Ölphase,
C) 0,1 - 1 Gew.-% Konservierungsmittel,
D) 0 - 2 Gew.-% Salz,
E) größer 80 Gew.-% Wasser.

Die Mikroemulsion selbst aber auch die feinteilige Emulsion liefern dann die hervorragenden Eigenschaften, die für eine Verwendung als Reinigungsmittel bevorzugt als Tränkungsmittel auf Flächengebilden wie Tüchern gefordert wird. Je nach Anforderungsprofil kommt die Mikroemulsion direkt zum Einsatz oder wird mit weiteren Zusatzstoffen als feinteilige Emulsion eingesetzt. Die feinteilige Emulsion kann stabilisiert sein durch den Zusatz von **Salz**. Das Salz zur Stabilisierung kann jedes Alkali- oder Erdalkalisalz sein, bevorzugt sind Halogenide, insbesondere Alkali- oder Erdalkalichloride, besonders bevorzugt ist Natriumchlorid. Es wurde gefunden, dass der Zusatz von Salz, bevorzugt Natriumchlorid, die positiven Eigenschaften der dünnflüssigen feinteiligen Emulsion nicht zerstören sondern stabilisierend auf die Zubereitung wirken. Es ist oftmals schwierig dünnflüssige Emulsionen, die durch Verdünnung aus Mikroemulsionen entstanden sind, so stabil zu formulieren, dass keine Entmischung trotz der niedrigen Viskosität auch über einen längeren Zeitraum stattfindet. Dies gelang durch den Zusatz der bevorzugten Mengen an Salz. Die Kontrolle wird durch optische Prüfung über vier Wochen vorgenommen. Entscheidend ist eine sichtbare Phasentrennung bei 5°C, Raumtemperatur und bei 40°C. Bei den erfindungsgemäßen Mikroemulsionen und auch bei den feinteiligen Emulsionen die NaCl enthalten, konnte auch nach vier Wochen bei den angegebenen Temperaturen noch keine Phasentrennung festgestellt werden.

Im Sinne der Erfindung bedeutet "**feinteilig**" eine nanoskalige mittlere Teilchengröße, speziell 5 bis 100 nm für die Teilchen in der Mikroemulsion und bei Verdünnung der Mikroemulsion eine Teilchengröße ≤ 1000 nm, besonders bevorzugt ≤200 nm. Die Teilchengröße wird bestimmt nach der DLS Methode mit einem Gerät der Bezeichnung Horiba LB-500.
Im Sinne der Erfindung versteht man unter "**niedrigviskos**" eine Viskosität, die ein Versprühen der Emulsionen mit üblichen Apparaturen ermöglicht. In der Regel handelt es sich dabei um Emulsionsviskositäten von ≤ 4000 mPas (Brookfield, Spindel 2, 20 rpm), vorzugsweise ≤ 2500 mPas, und besonders bevorzugt ≤ 500 mPas.

### Mikroemulsion

Unter Mikroemulsionen werden zunächst alle makroskopisch homogenen, optisch transparenten, niedrigviskosen und insbesondere thermodynamisch stabilen Mischungen aus zwei miteinander nicht mischbaren Flüssigkeiten und mindestens einem nichtionischen oder einem ionischen Tensid verstanden. Die mittleren Teilchengrößen der Mikroemulsionen liegen üblicherweise unter 100 nm, sie weisen eine hohe Transparenz auf und sind beim Zentrifugieren bei 2000 UPM für mindestens 30 Minuten gegenüber einer sichtbaren Phasenseparation stabil.

Die Herstellung der Mikroemulsionen in Schritt 1 erfolgt vorzugsweise einfach durch Vermischen der Ölphase mit den weiteren öllöslichen Inhaltsstoffen, Erwärmen der Ölphase über den Schmelzpunkt aller Bestandteile und anschließender Zugabe der wässrigen tensidhaltigen Phase. Die thermodynamisch stabile Mikroemulsion bildet sich dann spontan, ggf. muss noch etwas gerührt werden.

### APGs

Die Mikroemulsion enthält als zwingende Bestandteile eine Mischung von Zuckertensiden, und zwar Akyl(oligo)glycoside. Im Sinne der Erfindung wir der Begriff Alkyl(olig)-glycoside synonym zu Alky(poly)glycosiden verwendet und auch unter der Abkürzung "APG" aufgeführt. Alkyl- und/oder Alkenyloligoglucoside im Sinne der vorliegenden Lehre folgen dabei der Formel R¹O-[G]ₚ in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligoglucoside. Die Indexzahl p in der allgemeinen Formel (I) gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. APGs sind in den Mikroemulsionen gemäß der vorliegenden Erfindung in Mengen zwischen 10 und 30 Gew.-%, jeweils bezogen auf die Gesamtmenge der Mikroemulsion enthalten. Besonders bevorzugt sind dabei Mengen im Bereich von 15 bis 25 Gew.-%.
Die Mischung aus Alkyl(oligo)glycosiden verschiedener Kettenlänge für R¹ ist bevorzugt, da sich mit der Mischung eine homogenere Mikroemulsion herstellen lässt. Insbesondere bevorzugt ist eine Mischung aus APGs mit einer Kettenlänge für R¹ von 8-10 Kohlenstoffatomen in Kombination mit APGs der Kettenlänge für R¹ von 10-12 Kohlenstoffatomen.

### Cotensid:

Weiterhin sind Cotenside in der Mikroemulsion enthalten, die ausgewählt sind aus der Gruppe, die gebildet wird von Polyolfettsäureester, Zuckerester, W/O Emulgatoren wie Sorbitanester, Sorbitolpartialester, Polysorbate, Polyglycerylester, Polyglycerylpartialester, ein- und/oder zweiwertige Alkohole mit einer Kettenlänge von 2 bis 10 Kohlenstoffatomen wie beispielsweise Ethanol, Propanol, Butanol, Pentanol, Hexanol, Heptanol, Oktanol, Nonanol, Decanol, Pentandiol, Hexandiol, Heptandiol und Oktandiol.

### Anionische Tenside:

Im Sinne der Erfindung zeichnen sich die eingesetzten anionischen Tenside durch Säurefunktionen aus, die ganz oder teilweise neutralisiert vorliegen können. Bevorzugt ist die Neutralisierung mit Alkali oder Erdalkaligegenionen, vorzugsweise Natrium- oder Kaliumgegenionen. Vorzugsweise ist das anionische Tensid vollständig neutralisiert. Sie werden vorteilhafterweise bereits als (teil-)neutralisierte Komponenten eingesetzt. Sie führen zu einer Verminderung der Grenzflächenspannung.

Typische Beispiele für **anionische Tenside** sind Seifen, Alkylbenzolsulfonate, Alkansulfonate, Olefinsulfonate, α-Methylestersulfonate, Sulfofettsäuren, Alkylsulfate, Fettsäureamidsulfate, Mono- und Dialkyl-sulfosuccinate, Mono- und Dialkylsulfosuccinamate, Sulfotriglyceride, Amidseifen, Ethercarbonsäuren und deren Salze, Fettsäureisothionate, Fettsäuresarcosinate, Fettsäuretauride, N-Acylaminosäuren, wie beispielsweise Acyllactylate, Acyltartrate, Acylglutamate und Acylaspartate, Tatrate, Malonate, Malata, Citrate wie beispielsweise Glyceryl-Stearat-Citrat, Alkyloligoglucosidsulfate, Alkyloligoglucosidcarboxylate, Proteinfettsäurekondensate (insbesondere pflanzliche Produkte auf Weizenbasis), Alkylphosphate, Carboxylate. Besonders bevorzugt sind N-Acylaminosäuren, Succinate und Sulfosuccinate. Insbesondere bevorzugt sind Sodium-Stearyl-Glutamate und Disodium-Cetearyl-Sulfosuccinat.

### Organische Ölphase

Schließlich enthalten die Mikroemulsionen noch eine organische Ölphase, also eine nichtwasserlösliche organische Phase in Mengen von 10 bis 30 Gew.-%. Dabei sind besonders bevorzugte organische Ölphasen, ausgenommen alkoxylierte Verbindungen ausgewählt aus der Gruppe, die gebildet wird von Guerbetalkoholen auf Basis von Fettalkoholen mit 6 bis 20 C-Atomen, Estern linearer C₆-C₂₂-Fettsäuren mit linearen oder verzweigten C₆-C₂₂-Fettalkoholen bzw. Estern von verzweigten C₆-C₁₃-Carbonsäuren mit linearen oder verzweigten C₆-C₂₂-Fettalkoholen, Ester von linearen C₆-C₂₂-Fettsäuren mit verzweigten Alkoholen, Estern von C₆-C₂₂-Fettalkoholen und/oder Guerbetalkoholen mit aromatischen Carbonsäuren, Triglyceriden auf Basis C₆-C₁₀-Fettsäuren, Estern von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzlichen Öle, verzweigten primäre Alkoholen, substituierte Cyclohexanen, linearen und verzweigten C₆-C₂₂-Fettalkoholcarbonaten, Guerbetcarbonaten auf Basis von Fettalkoholen mit 6 bis 18, vorzugsweise 8 bis 10 C Atomen, Estern der Benzoesäure mit linearen und/oder verzweigten C₆-C₂₂-Alkoholen n linearen oder verzweigte, symmetrischen oder unsymmetrischen Dialkylethern mit 6 bis 22 Kohlenstoffatomen pro Alkylgruppe, und/oder aliphatischen bzw. naphthenischen Kohlenwasserstoffe, Dialkylcyclohexanen.

Als Ölphase können jedoch auch feste Fette und/oder Wachse eingesetzt werden. Diese können auch in Mischung mit den im vorherigen Abschnitt genannten Ölen vorliegen. Typische Beispiele für Fette sind Glyceride, d.h. feste oder flüssige pflanzliche oder tierische Produkte, die im Wesentlichen aus gemischten Glycerinestern höherer Fettsäuren bestehen. Ausgenommen werden jedoch die festen Monoglyceride wie z.B. Glycerinmonooleat oder Glycerinmonostearat da sie in der Mischung mit kosmetischen Zusatzstoffen wie Parfum oft zu Mischungsproblemen führen können oder zur Zersetzung der Mikroemulsion nach Verdünnung. Als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Tocopherole und ätherische Öle eignen sich ebenfalls als Ölkomponente.
Als Kohlenwasserstoffe werden organische Verbindungen bezeichnet, die nur aus Kohlenstoff und Wasserstoff bestehen. Sie umfassen sowohl cyclische als auch acyclische (=aliphatische) Verbindungen. Sie umfassen sowohl gesättigte wie einfach oder mehrfach ungesättigte Verbindungen. Die Kohlenwasserstoffe können linear oder verzweigt sein. Je nach Anzahl der Kohlenstoffatome im Kohlenwasserstoff kann man die Kohlenwasserstoffe einteilen in ungradzahlige Kohlenwasserstoffe (wie beispielsweise Nonan, Undecan, Tridecan) oder geradzahlige Kohlenwasserstoffe (wie beispielsweise Octan, Dodecan, Tetradecan). Je nach Art der Verzweigung kann man die Kohlenwasserstoffe einteilen in lineare (= unverzweigte) oder verzweigte Kohlenwasserstoffe. Gesättigte, aliphatische Kohlenwasserstoffe werden auch als Paraffine bezeichnet.
Besonders bevorzugte Ölphasen sind Esteröle wie Isopropylpalmitat, Isopropylmyristat, Ethylhexylpalmitat, Ethylhexylstearate, Di-n-Octylcarbonate, Dicaprylylcarbonate, Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat, Dioctyl Malate, Propylenglycol, Dimerdiol oder Trimertriol und Etheröle wie Dicaprylylether oder deren Mischungen.

Die weitere Ölphase, die in der feinteiligen Emulsion eingerührt wird, ist bevorzugt ausgewählt aus den im vorherigen Abschnitt genannten Esterölen und Etherölen wobei die Ölphase in der Mikroemulsion identisch oder verschieden zur weiteren Ölphase in der feinteiligen Emulsion sein kann. Bevorzugt ist die weitere Ölphase in der feinteiligen Emulsion verschieden zu der Ölphase in der enthaltenden Mikroemulsion. Erfindungsgemäß bevorzugt sind dabei homogene und klare Ölphasen.

### Fettalkohole

Neben den oben beschriebenen Inhaltsstoffen können die Mikroemulsionen als zusätzlichen Bestandteil noch Fettalkohole der allgemeinen Formel R²-OH enthalten, wobei R² für einen gesättigten oder ungesättigten, verzweigten oder unverzweigten Alkyl- oder Alkenylrest mit 6 bis 22 C-Atomen steht. Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind technische Fettalkohole mit 12 bis 18 Kohlenstoffatomen, wie beispielsweise Kokos-, Palm-, Palmkern- oder Talgfettalkohol. Besonders bevorzugt ist die Mitverwendung von Cetylalkohol, Stearylalkohol, Arachylalkohol und Behenylalkohol sowie deren Mischungen.
Wenn Fettalkohole enthalten sind werden sie bevorzugt in Mengen von 4 bis 10 Gew.-%, bezogen auf die gesamte Mikroemulsion eingesetzt, wobei der Bereich von 1 bis 5 Gew.-% besonders bevorzugt ist. Auch diese Fettalkohole, die wasserunlösliche organische Bestandteile darstellen, fallen erfindungsgemäß nicht unter die Definition der Ölphase.

### Kosmetische Zusatzstoffe:

Die feinteilige Emulsion kann weiterhin noch übliche kosmetische Hilfs- und Zusatzstoffe enthalten, die dem Fachmann bekannt sind wie beispielsweise Konsistenzgeber, anorganische und organische UV-Lichtschutzfilter, Selbstbräuner, Pigmente, Antioxidationsmittel, Hydrotope, Deodorant- und Antitranspirantwirkstoffe, biogene Wirkstoffe, Farbstoffe, zusätzliche Konservierungsmittel bevorzugt Benzoesäure oder Zitronensäure, Feuchthaltemittel wie Glycerin und Parfums. Als biogene Wirkstoffe sind dabei insbesondere Tocopherol, Tocopherolacetat, Tocopherolpalmitat, Desoxyribonucleinsäure, Coenzym Q10, Ascorbinsäure, Retinol- und Retinylderivate, Bisabolol, Allantoin, Phytantriol, Panthenol, AHA-Säuren, Aminosäuren, Ceramide, essentielle Öle, Hyaloronsäure, Creatin, Proteinhydrolysate, Pflanzenextrakte, Peptide und Vitaminkomplexe bevorzugt.

### Wasser

Ein weiterer wesentlicher Bestandteil der Mikroemulsionen ist Wasser. Das Wasser sollte vorzugsweise demineralisiert sein. Die Mikroemulsionen enthalten vorzugsweise bis zu 80 Gew.-% Wasser. Bevorzugte Bereiche für den Wasseranteil in der Mikroemulsion sind Mengen von 20 bis 80 Gew.-% und insbesondere von 30 bis 65 Gew.-% Wasser in der Mikroemulsion. Für die feinteilige Emulsion ergibt sich ein bevorzugter Wasseranteil von größer 80 Gew.-% bezogen auf die Gesamtmenge der feinteiligen Emulsion. Dies bedeutet, dass in den 80 Gew.-% der Anteil an Wasser aus der enthaltenden Mikroemulsion einbezogen ist. Ebenso ist Wasser aus den anderen Inhaltstoffen, die nie frei von Wasser sind, mit einbezogen.

### Verwendung

Ein weiterer Gegenstand der Erfindung ist die Verwendung der erfindungsgemäßen Mikroemulsion als kosmetisches Reinigungsmittel in Tüchern oder Wipes, Duschgelen, Handwaschmitteln, Gesichtsreinigern, make-up-Entfernern, Badezubereitungen, Babypflegeprodukten, Shampoo, Hautreinigungsmitteln und ähnliches.
Des weiteren ist folgerichtig ein weiterer Gegenstand der Erfindung die Verwendung der erfindungsgemäßen feinteiligen Emulsionen als kosmetisches Reinigungsmittel in Tüchern oder Wipes, Duschgelen, Handwaschmitteln, Gesichtsreinigern, make-up-Entfernern, Badezubereitungen, Babypflegeprodukten, Shampoo Hautreinigungsmitteln und ähnliches.
Des weiteren ist weiterhin folgerichtig ein weiterer Gegenstand der Erfindung die Verwendung der erfindungsgemäßen feinteiligen Emulsionen als Tränkungsmedium für Tücher und/oder Gewebe. Je nach Beschaffenheit der Tücher und Gewebe die zur Reinigung verwendet werden sollen, kann es auch möglich sein die erfindungsgemäße Mikroemulsion direkt als Tränkungsmittel zu nutzen. Die niedrige Viskosität lässt ein Aufsprühen oder Tränken zu. Dementsprechend kann sowohl die Mikroemulsion als auch die feinteilige Emulsion verwendet werden als sprühbare Zubereitung in Sprühapplikation zu Reinigungszwecken. Als weiterer Gegenstand der Erfindung sind demnach auch die kosmetischen Tücher, die entweder mit der Mikroemulsion oder mit der erfindungsgemäßen feinteiligen Emulsion benetzt oder getränkt sind. Die Tücher können gepresst oder gewebt vorliegen, sie können dabei feucht oder trocken angeboten werden. Im Sinne der Erfindung werden die Bezeichnungen Tücher, Reinigungs- und Pflegetücher, Wipes oder Flächengewebe und Flächengebilde synonym verwendet. Die erfindungsgemäße Mikroemulsion und die erfindungsgemäße feinteilige Emulsion eignen sich auch sowohl zur Herstellung von Reinigungs- und Pflegetüchern als auch zur direkten Anwendung in Form sprühbarer Emulsionssysteme zur Reinigung und Pflege von Oberflächen in Haushalt und Industrie wie etwa der Textilpflege, der Lederpflege, der Pflege und Reinigung von metallischen und nichtmetallischen Oberflächen. Insbesondere steht dabei der Einsatz als Tränklösung zur Herstellung von Feuchttüchern sowie die Verwendung in sprühbaren Zubereitungen im Vordergrund.

### Verfahren

Die erfindungsgemäßen Mikroemulsionen sind herstellbar unter Verwendung eines einfachen Rührwerkzeuges. Die Herstellung der Mikroemulsion erfolgt vorzugsweise einfach durch Vermischen der Ölphase mit den weiteren öllöslichen Inhaltstoffen, Erwärmen der Ölphase über den Schmelzpunkt aller Bestandteile und anschließender Zugabe der wässrigen tensidhaltigen Phase. Die thermodynamisch stabile Mikroemulsion bildet sich dann spontan, ggf muss noch etwas gerührt werden. Unter Zugabe von Zitronensäure wird der pH-Wert auf 3,0 bis 7,0 eingestellt.
Als weiterer Gegenstand der Erfindung kann in einem zweiten Schritt diese Mikroemulsion mit den Bestandteilen der erfindungsgemäßen feinteiligen Emulsion vermischt werden. Dazu wird zunächst die Mikroemulsion mit dem 5 bis 20 fachen Volumen der Mikroemulsion bei Temperaturen von 15 bis 35°C mit Wasser verdünnt und anschließend die Komponenten B bis E unter Rühren zugefügt.
A) Mikroemulsion
B) gegebenenfalls eine weitere Ölphase
C) Konservierungsmittel
D) Gegebenenfalls Salz
E) Wasser (größer 80 Gew.-%) bezogen auf die gesamte feinteilige Emulsion.

### Beispiele

### 1. Die erfindungsgemäße Mikroemulsion - Beispielformulierung 1 bis 7

Die Konzentrationsangaben in allen Beispielen sind als Gewichtsprozente (Gew.-%) bezogen auf die gesamte Mikroemulsion bzw. feinteilige Emulsion angegeben.
Alle Substanzen sind Produkte der Firma Cognis. Alle Substanzbezeichnungen sind eingetragene Marken.

**Tabelle 1: Rezepturen für Mikroemulsionen**

| **Substanz** | **INCI** | **Gew.-% Aktivsubstanz** | | | | | | |
|---|---|---|---|---|---|---|---|---|
| | | **1** | **2** | **3** | **4** | **5** | **6** | **7** |
| Plantacare 1200 UP | Lauryl Glycoside | 6,8 | 4,1 | 12,6 | 11,3 | 11,3 | 7 | 7 |
| Plantacare 810 UP | Decyl Glycoside | 6,8 | 9,5 | 8,5 | 7,4 | 8,6 | 12 | 12 |
| Dehymuls SSO | Sorbitan Sesquioleate | 4,2 | | 5,0 | 6,0 | 6,2 | 5,0 | 5,6 |
| Dehymuls SMO | Sorbitan Oleate | | 4,7 | | | | | |
| Lameform TGI | Polyglyceryl-3-Diisostearate | 4,0 | 4,5 | 4,1 | 4,1 | 5,0 | | 4,2 |
| Eumulgin SG | Sodium Stearyl Glutamate | 1,5 | 1,0 | | | | | |
| Eumulgin Prisma | Disodium-Cetearyl-Sulfosuccinate | | | 0,9 | 1,1 | 1,3 | 1,5 | 1,6 |
| Cetiol OE | Dicaprylylether | 10,0 | 7,0 | 9,9 | 10,0 | 12,0 | 11,2 | 11 |
| IPP | Isopropylpalmitate | 10,0 | 13,0 | 5,4 | 6,6 | 6,5 | 6 | 6 |
| | Glycerin | | | 3 | 3 | 3 | 8 | 8 |
| Lanette O | Cetearyl Alcohol | | | | | | 2 | |
| | Zitronensäure-Lsg. (50%) | q.s. | q.s | q.s. | q.s. | q.s. | | |
| | Benzoesäure | 0,5 | 0,5 | 0,5 | 0,5 | 0,5 | 0,5 | 0,5 |
| | Wasser | add 100 | add 100 | add 100 | add 100 | add 100 | add 100 | add 100 |

Die Mikroemulsion wurde hergestellt durch Vermischen der Komponenten unter Rühren bei einer Temperatur von 75 °C. Anschließend auf Raumtemperatur abkühlen lassen. Durch die Zugabe von Zitronensäure-Lösung (50%) wurde der pH-Wert eingestellt zwischen 3,0 und 7,0.
Die Teilchengröße wurde bestimmt mit einem Gerät Horiba LB-500 (Methode: DLS) und betrug nach Verdünnung auf 2 % Aktivsubstanz 30 bis 100 nm. Die Viskosität wurde mit einem Brookfield Viskosimeter, Spindel 2, bei 20 rpm bestimmt und betrug < 300 mPas.

### 2. Feinteilige Emulsion / Effekt von Salzzugabe

Zur Herstellung der feinteiligen Emulsion kann diese Mikroemulsion beispielsweise in einem weiteren Schritt in folgende Rezeptur eingemischt werden. Das Ziel klare, bläuliche feinteilige Emulsionen zu bilden wird z.T. nur durch die Zugabe von Salz erreicht.

**Tabelle 2: Rezepturen für feinteilige Emulsionen**

| | **Substanz** | **INCI** | **1** | **2** | **3** | **4** | **5** | **6** |
|---|---|---|---|---|---|---|---|---|
| 1 | Mikroemulsion (Tabelle 1) | | 4,5 | 10,0 | 4,5 | 4,5 | 4,5 | 4,5 |
| 2 | Cetiol CC | Dicarprylyl Carbonate | | | 0,5 | 0,5 | | |
| 3 | Cetiol OE | Dicaprylylether | | | | | 0,5 | 0,5 |
| 4 | Copherol F 1300 C | Tocopherol | | | | | | |
| 5 | D- Panthenol | | | | | | | |
| 6 | Perfume | Perfume | | 0,1 | 0,1 | 0,1 | 0,1 | 0,1 |
| 7 | Phenoxyethanol | Phenoxyethanol | | | 1,0 | 1,0 | 1,0 | 1,0 |
| 8 | Euxyl K 702 | *** | | | | | | |
| 9 | Natrium Benzoat | | 0,5 | 0,5 | 0,5 | 0,5 | 0,5 | 0,5 |
| 10 | Na Cl | | | | | 0,5 | | 0,5 |
| 11 | KOH (20%ig) | | | | | | | |
| 12 | Wassser | | ad 100,0 | ad 100,0 | ad 100,0 | ad 100,0 | ad 100,0 | ad 100,0 |
| | pH -Wert | | 4,9 | 4,8 | 4,9 | 4,8 | 4,9 | 4,7 |
| | Stabilität nach Tag 1 bei Raumtemperatur | | stabil | stabil | instabil | stabil | stabil | stabil |
| | Stabilität nach 4 Wochen bei Raumtemperatur | | stabil | stabil | instabil | stabil | instabil | stabil |

| | **Substanz** | **INCI** | **7** | **8** | **9** | **10** | **11** | |
|---|---|---|---|---|---|---|---|---|
| 1 | Mikroemulsion (Tabelle 1) | | 4,5 | 10,0 | 4,5 | 10,0 | 10,0 | |
| 2 | Cetiol CC | Dicarprylyl Carbonate | | | 0,3 | | 0,3 | |
| 3 | Cetiol OE | Dicaprylylether | | | | | | |
| 4 | Copherol F 1300 C | Tocopherol | 0,1 | | | | | |
| 5 | D- Panthenol | | | 0,5 | 0,5 | | | |
| 6 | Perfume | Perfume | 0,1 | 0,1 | 0,1 | 0,1 | 0,1 | |
| 7 | Phenoxyethanol | Phenoxyethanol | 1,0 | 1,0 | 1,0 | | | |
| 8 | Euxyl K 702 | *** | | | | 1,0 | 1,0 | |
| 9 | Natrium Benzoat | | 0,5 | 0,5 | 0,5 | | | |
| 10 | Na Cl | | | | | | | |
| 11 | KOH (20%ig) | | | | | 0,8 | 0,8 | |
| 12 | Wassser | | ad 100,0 | ad 100,0 | ad 100,0 | ad 100,0 | ad 100,0 | |
| | pH -Wert | | 4,9 | 4,6 | 4,8 | 4,9 | 4,9 | |
| | Stabilität nach Tag 1 bei RT | | stabil | stabil | stabil | stabil | stabil | |
| | Stabilität nach 4 Wochen bei RT | | stabil | stabil | stabil | stabil | stabil | |

| | | | | | | | | |
|---|---|---|---|---|---|---|---|---|
| *** Phenoxyethanol, Benzoic Acid, Dehydroacetic Acid, Aqua, Ethylhexylglycerin, Polyaminopropyl Biguanide Alle Angaben in Gew.% Aktivsubstanz bezogen auf das Gesamtgewicht der Zubereitung. | | | | | | | | |

Die feinteiligen Emulsionen wurden hergestellt, indem zunächst Wasser vorgelegt wurde, anschließend das Natriumbenzoat und gegebenenfalls das Natriumchlorid und/ oder wasserlösliche Actives (D-Panthenol) unter Rühren zugegeben wurden und dann ein Premix aus öllöslichen Substanzen (1, 2, 3, 4, 6, 7, 8) eingerührt wurde bis eine homogene Verdünnung entstand. Falls notwendig wird der pH-Wert eingestellt auf pH 4,5 -5,2. Die Stabilität wurde durch optische Prüfung bestimmt.

## Patentansprüche

1. Eine Mikroemulsion enthaltend:
a1) eine Mischung aus Alkyl(oligo)glycosiden,
a2) mindestens ein Cotensid,
a3) mindestens ein anionisches Tensid, das sich auszeichnet durch Säurefunktionen, die ganz oder teilweise neutralisiert vorliegen können,
a4) eine organische Ölphase ausgenommen alkoxylierte Verbindungen,
a5) gegebenenfalls Fettalkohole ausgenommen alkoxylierte Fettalkohole,
a6) gegebenenfalls kosmetische Zusatzstoffe,
a7) Wasser add 100 Gew.%.

2. Eine Mikroemulsion nach Anspruch 1, enthaltend
a1) 10 - 30 Gew.-% einer Mischung aus Alkyl(oligo)glycosiden,
a2) 2 - 15 Gew.-% Cotenside,
a3) 0,2 - 4 Gew.-% mindestens eines anionischen Tensides,
a4) 10 - 30 Gew.% organische Ölphase ausgenommen alkoxylierte Verbindungen,
a5) 0 - 4 Gew.-% Fettalkohole ausgenommen alkoxylierte Fettalkohole,
a6) 0 - 20 Gew.-% kosmetische Zusatzstoffe,
a7) Wasser add 100 Gew.-%.

3. Eine Mikroemulsion nach Anspruch 1 oder 2, **dadurch gekennzeichnet, dass**
a1) die Mischung aus Alkyl(oligo)glycosiden ausgewählt sind aus Verbindungen der allgemeinen Formel R¹O-[G]ₚ, in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht,
a2) die Cotenside ausgewählt sind aus der Gruppe, die gebildet wird aus Polyolfettsäureester, Zuckerester, W/O Emulgatoren wie Sorbitanester, Sorbitolpartialester, Polysorbate, Polyglycerylester, Polyglycerylpartialester, ein- und/oder zweiwertige Alkohole mit einer Kettenlänge von 2 bis 10 Kohlenstoffatomen,
a3) die anionischen Tenside ausgewählt sind aus der Gruppe, die gebildet wird von N-Acylaminosäuren, Citrate, Tartrate, Malate, Malonate, Succinate, Sulfosuccinate, Alkylsulfonate, Alkylsulfate oder Carboxylate,
a4) die organische Ölphase Verbindungen enthält, die ausgewählt sind aus der Gruppe, die gebildet wird von Fettalkoholethern, Fettsäureester langkettiger Fettalkohole, Dialkylcarbonate,
a5) die Fettalkohole ausgewählt sind aus der Gruppe mit der allgemeinen Formel R²-OH, wobei R² für einen gesättigten oder ungesättigten, verzweigten oder unverzweigten Alkyl- oder Alkenylrest mit 6 bis 22 C-Atomen steht,
a6) die kosmetischen Zusatzstoffe ausgewählt sind aus der Gruppe, die gebildet wird von Glycerin, Benzoesäure oder ihre Salze, Zitronensäure.

4. Feinteilige Emulsion enthaltend
A) eine Mikroemulsion nach mindestens einem der Ansprüche 1 bis 3,
B) gegebenenfalls eine weitere Ölphase,
C) Konservierungsmittel,
D) gegebenenfalls Salz,
E) Wasser (größer 80 Gew.-%) bezogen auf die gesamte feinteilige Emulsion.

5. Feinteilige Emulsion nach Anspruch 4, enthaltend
A) 0,5 - 15 Gew.-% einer Mikroemulsion nach Anspruch 1 bis 3,
B) 0 - 10 Gew.-% einer weiteren Ölphase,
C) 0,1 - 3 Gew.-% Konservierungsmittel,
D) 0 - 5 Gew.-% Salz,
E) größer 80 Gew.-% Wasser.

6. Verwendung von Mikroemulsionen nach einem der Ansprüche 1 bis 3 als kosmetisches Reinigungsmittel in Tüchern, Duschgelen, Handwaschmitteln, Gesichtsreinigern, make-up-Entfernern, Badezubereitungen, Babypflegeprodukten, Shampoo.

7. Verwendung von feinteiligen Emulsionen nach einem der Ansprüche 4 bis 5 als Tränkungsmedium für Tücher und/oder Gewebe.

8. Verwendung von feinteiligen Emulsionen nach einem der Ansprüche 4 bis 5 als kosmetisches Reinigungsmittel in Tüchern, Duschgelen, Handwaschmitteln, Gesichtsreinigern, make-up-Entfernern, Badezubereitungen, Babypflegeprodukten, Shampoo.

9. Kosmetische Tücher, **dadurch gekennzeichnet, dass** sie mit einer feinteiligen Emulsion gemäß einem der Ansprüche 4 bis 5 benetzt sind.

10. Verfahren zur Herstellung einer feinteiligen Emulsion nach einem der Ansprüche 4 bis 5, **dadurch gekennzeichnet, dass** man zunächst in einem ersten Schritt eine Mikroemulsion herstellt gemäß einem der Ansprüche 1 bis 3, in einem zweiten Schritt diese Mikroemulsion mit dem 5 bis 20 fachen Volumen der Mikroemulsion bei Temperaturen von 15 bis 35°C mit Wasser verdünnt und anschließend die Komponenten B bis E gemäß Ansprüche 4 bis 5 unter Rühren hinzufügt.
